# EUROPEAN PATENT SPECIFICATION

(11) **EP 3 801 219 B1**
(45) Date of publication and mention of the grant of the patent: **15.01.2025**
(21) Application number: 19812243.4
(22) Date of filing: 31.05.2019
(51) Int. Cl.: A61B 5/00, G01L 1/24, G01L 7/02, G01L 13/02, G01N 21/70, G01N 33/483, G06F 3/03, A61B 5/021, A61B 5/022

(54) **OPTICAL BLOOD PRESSURE MEASUREMENT DEVICE**
VORRICHTUNG ZUR OPTISCHEN BLUTDRUCKMESSUNG
DISPOSITIF DE MESURE OPTIQUE DE LA PRESSION ARTÉRIELLE

(30) Priority: 01.06.2018 US 201862679435 P
(43) Date of publication of application: 14.04.2021
(73) Proprietor: Cardio Ring Technologies, Inc., San Jose CA 95134 (US)
(72) Inventor: CHANG, Kuang-Fu, Taichung City, 406 (TW); WANG, Yu-Chi, Taipei, 106 (TW); CHEN, Leng-Chun, Hsinchu City, 30080 (TW); CHEN, Jun-Ming, Bethesda, Maryland 20852 (US); SHIH, Wen-Pin, Taipei, 100 (TW)
(74) Representative: Thoma, Michael
(86) International application number: PCT/US2019/034856
(87) International publication number: WO 2019/232334

(56) References cited:
- US-A1- 2008 076 981
- US-A1- 2008 091 113
- US-A1- 2009 315 989
- US-A1- 2011 063 257
- US-A1- 2011 152 694
- US-A1- 2015 177 909
- US-A1- 2018 049 655
- US-B2- 6 820 496
- WOLTHUIS R A ET AL: "DEVELOPMENT OF MEDICAL PRESSURE AND TEMPERATURE SENSORS EMPLOYING OPTICAL SPECTRUM MODULATION", IEEE TRANSACTIONS ON BIOMEDICAL ENGINEERING, IEEE, USA, vol. 38, no. 10, 1 October 1991 (1991-10-01), pages 974 - 981, XP000264291, ISSN: 0018-9294, DOI: 10.1109/10.88443
- LIPOMI ET AL.: "Skin-like pressure and strain sensors based on transparent elastic films of carbon nanotubes", NATURE NANOTECHNOLOGY, vol. 6, no. 12, 2011, pages 788 - 792, XP055390039, [retrieved on 20190912]

## Description

The present invention relates to devices for detecting a force in a surface region of tissue and measuring blood pressure.

### BACKGROUND OF THE INVENTION

Cardiovascular disease (CVD) accounts for approximately a significant number of deaths on a world-wide basis. CVD includes coronary heart disease (CHD), which accounts for the majority of CVD deaths, as well as stroke and heart failure. Many more individuals carry a diagnosis of CVD and live with the diagnosis. Those living with CVD are at risk of acute heart attack, strokes and other chronic conditions that can adversely affect the individual's quality of live over a long term long-term. Ultimately, CVD increases the risks of mortality in the patient. Therefore, there is a keen interest by governments, healthcare providers, as well as the general population to prevent CVD.

The rise of portable smart-devices, such as smart phones, smart watches, fitness monitors, etc. has given individuals a useful tool to monitor health parameters to address CVD symptoms, where such health parameters include blood pressure and heart rate. Such devices are also of interest to healthy individuals so that who can monitor such data to avoid the onset or progression of CVD. In addition, a number of drugs or therapeutic strategies treat or manipulate the cardiovascular diseases. As a result, predicting short-term and long-term risk of cardiovascular diseases for people plays an important role in treatment. To this end, although pathogenesis of different cardiovascular diseases might be distinct from each other, most of them can be monitored and precautionary assessed through specific physical signs. Since most cardiovascular diseases including hypertension diseases and hypotension diseases are significantly related to blood pressures and such monitoring techniques thereof are not well-developed and implemented universal, there is a need to establish or develop a monitoring device or a monitoring method for monitoring blood pressures in households or hospitals in a simpler manner.

Non-invasive blood pressure measuring devices including sphygmomanometers and photoplethysmography are used in monitoring patient' s blood pressures to prevent various cardiovascular diseases or provide doctors with early diagnosis. However, most of them are bulky and heavy which are inconvenient for outdoor applications and long-time monitoring.

Furthermore, a need remains for a device to monitor blood pressure but avoids discomfort for patients.

Previously, wearable blood-pressure monitoring devices that allowed for real-time monitoring and portable capability are described in US20180049655 and WO2018005298, the entirety of each of which is incorporated by reference. However, there remains a need to more accurately measure blood pressure using a portable, non-obtrusive device. Further devices are known from US 2015/177909 A1 and US 2011/063257 A1, wherein US 2015/177909 suggests deforming an optical thin film layer by means of applying a shear force via a fingertip, thereby creating a portion with increased thickness that can be determined by reflected light differing in the power spectrum.

### BRIEF SUMMARY OF THE INVENTION

The present disclosure includes a force detecting device that uses elastomeric polymers to determine application of a force applied to the device. In one variation, the present disclosure includes devices for detecting a force in a surface region of tissue. For example, the device includes a transparent backing material comprising a planar shape, the transparent backing material comprising a first surface and a second surface on an opposite side of the planar shape; a first elastomer on the first surface of the transparent backing material, where a light transmission property of the first elastomer changes upon application of force to the first elastomer; and wherein when positioned on the surface region of tissue the force in the surface region causes a deformation of the first elastomer resulting in a change in the light transmission property of the first elastomer.

The configuration described herein using polymers and optical devices allows for the optical devices to be monolithically integrated into a chip, allowing further miniaturization of the device. The configuration of the device allows for the optical devices could to be a camera module of a smart phone, allowing the user to measure their own blood pressure on demand. The configuration also allows this measurement method to be applied on other portions of a body rather than just on a digit.

The device further includes a second elastomer on the first surface of the transparent backing material, where a light transmission property of the second elastomer changes upon application of force to the first elastomer; an opaque divider between the first elastomer and the second elastomer to block propagation of light therebetween; a stiffening layer on the second elastomer on a side opposite to the transparent backing material; and wherein when positioned on the surface region the stiffening layer prevents the force from changing the light transmission property of the second elastomer such that the second elastomer provides a reference to determine a deformation of the first elastomer.

A variation of the device further includes an opaque cover on the first elastomer and the second elastomer located on the side opposite to the transparent backing material, where the stiffening layer is located on the opaque layer and adjacent to the second elastomer.

Another variation of the invention includes an opaque cover on the first elastomer and the second elastomer located on the side opposite to the transparent backing material, where the stiffening layer is located on the second elastomer and opaque layer and adjacent to the opaque cover. Variations of the device can include a stiffening layer, additive, or reinforcement on any portion of the elastomers.

The device further includes a light emitting source and a light detecting element both located adjacent to the first elastomer and possibly to the second elastomer, where the light emitting source is configured to illuminate the first elastomer and possibly the second elastomer and where the light detecting element is configured to determine an absorption of light in the first elastomer and possibly in the second elastomer.

Another variation of the device includes the light detecting element being configured to transmit a signal to a controller, where the signal comprises data of the absorption of light in the first elastomer and possibly in the second elastomer to determine the force in the surface region.

The present disclosure also includes method of measuring a blood pressure in an artery within a region of tissue. The measurements can be continuous over a period of time or on demand. The method includes positioning a device as described above on the region of tissue, where deformation of the region of tissue causes deformation of the first elastomer; illuminating the first elastomer; observing an emission of light from the first elastomer during application of a force on the first elastomer where the force is produced by the artery; and determining a change in the emission of light caused by application of the force to calculate a blood pressure in the artery.

A variation of the method can include a second elastomer, where the second elastomer is configured such that deformation of the region of tissue does not cause deformation of the second elastomer. The method can include illuminating the second elastomer during illuminating of the first elastomer.

A variation of the method further includes observing an emission of light from the second elastomer during application of the force on the first elastomer.

The methods can include comparing the emission of light of the first elastomer to the emission of light from the second elastomer.

The methods described herein can be performed on a region of tissue such as a digit, an arm, a leg, or any body part where measurement of tissue displaced by blood flow in an artery occurs.

The methods and device discussed herein can transmit the blood pressure information via a wired or wireless connection to any personal electronic device including but not limited to a smart phone, a smart watch, a fitness tracker, a tablet, a computer, and/or a network.

The methods and devices can also continuously illuminate the first elastomer for a period of time to continuously calculate the blood pressure in the artery over the period of time.

Another variation of the devices described herein include a patch that converts external forces into change of light absorption, comprising: a transparent backing; a light-absorptive sensing elastomer on one surface of the transparent backing, wherein: the light absorption of the light-absorptive sensing elastomer is indicative of the elastomer deformation subjected to static and fluctuating external forces.

The patch can further include an opaque cover on the surface, opposite to the interface between the transparent backing and the light-absorptive sensing elastomer, of the light-absorptive sensing elastomer.

A variation of the patch further includes a light-absorptive reference elastomer on the surface of the transparent backing and by one side of the light-absorptive sensing elastomer, wherein: the light absorption of the light-absorptive reference elastomer is indicative of the elastomer deformation subjected to static external forces.

The patch can also include an opaque divider that prohibits light propagation between the light-absorptive reference elastomer and the light-absorptive sensing elastomer.

Additional variations of the patch include an opaque cover on the surface, opposite to the interface between the transparent backing and the light-absorptive sensing elastomer, of the light-absorptive sensing elastomer and the light-absorptive reference elastomer.

The present disclosure also includes methods to measure blood pressure. For example, such a method can include attaching a patch, that converts external forces into change of light absorption, on the skin under which an artery passes through; emitting at least a light into the patch; measuring the lights propagating out from the patch; and converting the measurement of the lights, propagating out from the patch, into blood pressure.

The disclosure also includes variations of continuous blood pressure monitoring systems. For example, such systems include a patch that converts external forces into change of light absorption; a light emitter that emits at least a light into the patch; a light detector that measures the lights propagating out from the patch; and an algorithm that converts the measurement of the lights, propagating out from the patch, into blood pressure.

A variation of the continuous blood pressure monitoring system includes a transparent backing; and a light-absorptive sensing elastomer on one surface of the transparent backing, wherein: the light absorption of the light-absorptive sensing elastomer is indicative of the elastomer deformation subjected to static and fluctuating external forces.

The present disclosure also includes wearable devices that continuously monitor blood pressure. Such devices include a ring body; a light emitter disposed on a monitoring surface at the inner side of the ring body; a light detector disposed on a monitoring surface at the inner side of the ring body and by a side of the light emitter; and a light-absorptive sensing elastomer covering the light emitter and the detector, wherein: the light absorption, which is measured by the light detector, of the light-absorptive sensing elastomer is indicative of the blood pressure of a wearer.

Another example of a wearable device that continuously monitors blood pressure, includes a ring body; a light emitter disposed on a monitoring surface at the inner side of the ring body; a light detector disposed on a monitoring surface at the inner side of the ring body and by a side of the light emitter; a light-absorptive sensing elastomer covering a portion of the light emitter and a portion of the detector; and a light absorptive reference elastomer covering the remaining portion of the light emitter and the remaining portion of the detector; wherein: the comparative light absorption, which is measured by the light detector, of the light-absorptive sensing elastomer and the light-absorptive reference elastomer is indicative of the blood pressure of a wearer.

### BRIEF DESCRIPTION OF THE SEVERAL VIEWS OF THE DRAWINGS

FIGS. 1A and 1B illustrate respective front and oblique views of an example of a device configured to monitor blood pressure using movement in tissue that is driven by the blood flow within a vessel located in that tissue.
FIGS. 2A and 2B illustrate additional variations of tissue displacement assemblies for use with additional variations of blood pressure measuring devices.
FIG. 3A illustrates components of a force detecting device used to detect movement.
FIG. 3B shows components of another variation of a force detecting device where a second or reference elastomer is positioned adjacent to a first elastomer.
FIG. 4A illustrates another variation of components of a variation of a force detecting device showing a first elastomer and a second elastomer on a transparent backing material where the elastomers are separated by an opaque divider.
FIG. 4B illustrates a cross sectional view of the components shown in FIG. 4A taken along the line 4B-4B to demonstrate an operation of a variation of a force detecting device.
FIG. 5A illustrates a variation of a force detecting device positioned on a finger to detect a blood pressure of a vessel within the finger using displacement of tissue adjacent to the blood vessel.
FIG. 5B shows a cross sectional view of the device of FIG. 5A taken along line 5B-5B.
FIG. 5C is a cross sectional illustration taken along lines 5C-5C from FIG. 5A.
FIGS. 6A to 6D illustrate another variation of a device having a force detecting apparatus positioned on a ring body.

### DETAILED DESCRIPTION OF THE INVENTION

Methods and devices are described herein that relate to monitoring blood pressure in a vessel of a region of tissue. The methods and devices described herein can monitor blood pressure in a digit of a hand or in other areas of the body where the pulsatile flow of blood in a vessel displaces adjacent tissue that can be detected from a surface of the tissue. In addition, the methods and devices disclosed herein include improvements for detecting movement in a tissue of a region of the body, where the movement in the tissue arises from blood pressure changes within a vessel in that tissue. Optionally, the devices and methods described herein can be used wearable devices and non-invasive monitoring blood-pressure in real-time.

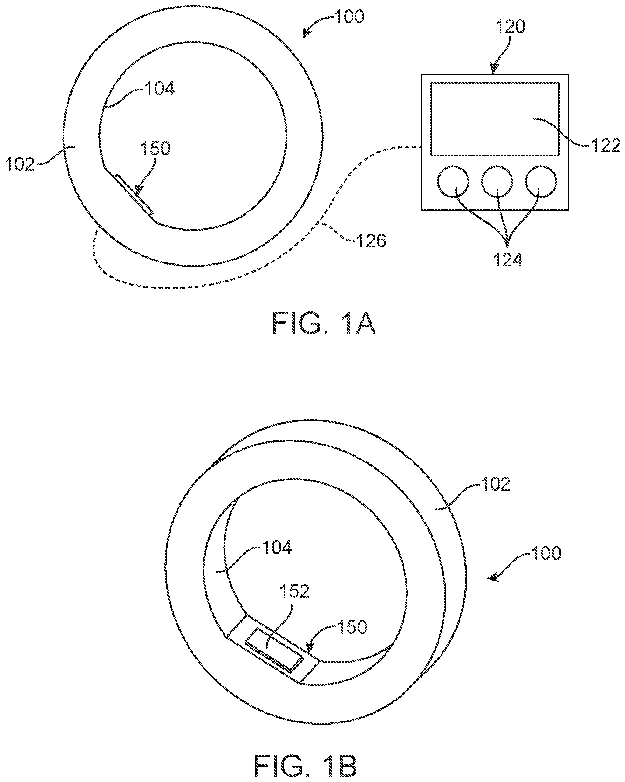

FIGS. 1A and 1B illustrate respective front and oblique views of an example of a monitoring device 100 configured to monitor blood pressure using movement in tissue that is driven by the blood flow within a vessel located in that tissue. In the examples illustrated in FIGS. 1A and 1B, the variation of the monitoring device 100 is configured with a ring-shaped body 102 that houses a device for detecting a force in a region of tissue 150 where a portion of the force detecting device 150 protrudes from an inner surface 104 of the ring shaped body 100. This variation is suited for placement about a digit of an individual's hand such that it can detect movement of tissue in the digit that is caused by pulsatile flow of a vessel within the digit/tissue. However, additional variations of a blood pressure monitoring apparatus under the present disclosure are not limited to ring-type devices. The movement detecting apparatus 150 monitors movement of tissue in the digit, where the tissue movement can be caused by the oscillation of the blood vessel due to pressure changes therein. As shown, the device 100 can communicate 126 (either via a wire, wireless connection, cloud-based transmission, etc.) to a user interface 120. The user interface 120 can comprise a body wearable apparatus or can comprise a computer, smart-phone, smart-watch, tablet, or other electronic apparatus. Variations of the user interface 120 can include a feedback portion 122 (either visual, audible, etc.) and/or controls 124.

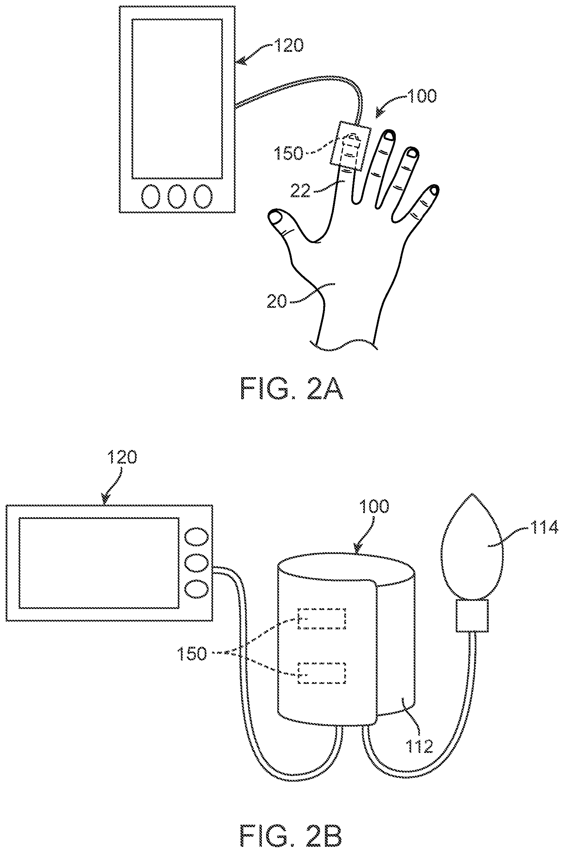

FIGS. 2A and 2B illustrate additional variations of tissue displacement assemblies 150 for use with additional variations of blood pressure measuring devices 100. The variation shown in FIG. 2A illustrates a finger cuff or cradle 110 that houses one or more force detecting devices 150. As shown, a finger 22 of a hand 20 is positioned within or on the device 110 such that one or more force detecting assemblies 150 can detect movement of tissue and transmit information (via a wired or wireless connection 126) to a user interface device 120 (as described above) such that blood pressure can be monitored. FIG. 2B illustrates a traditional blood pressure cuff 122 having a pump or bladder 114 that is used to secure the cuff 112 about a leg or arm of a patient. The cuff 112 includes any number of force detecting devices 150 that can measure displacement of tissue due to pulsatile flow of blood in a vessel within the tissue that is adjacent to the cuff 112.

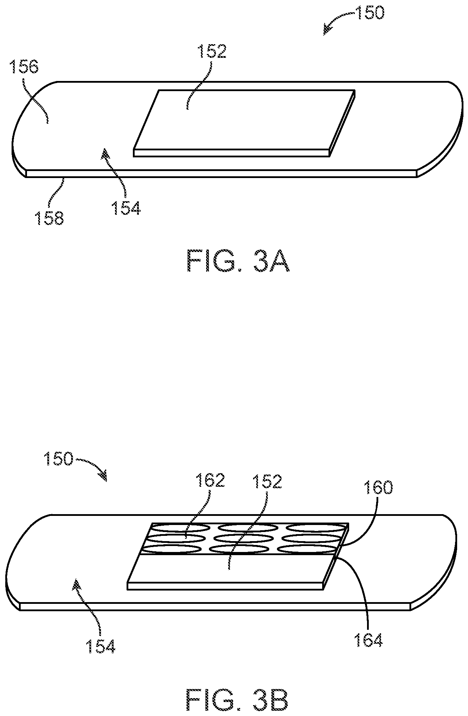

FIG. 3A illustrates components of a force detecting device 150 used to detect movement. Variations of the device 150 can be used as described herein to monitor movement of tissue arising from flow of blood in an artery within the tissue. The force detecting device 150 includes a first elastomer 152 a first surface 156 of a transparent backing material 154, where a light transmission property of the first elastomer 152 changes upon application of force to the first elastomer 152. The first elastomer 152 and the second elastomer 160 can comprise the same or different materials. However, as noted below, the operation of the first and second elastomers 152 160 will vary in the device 150.

In this variation of the device 150, the first surface 156 of the transparent backing material 154 is positioned facing tissue while the second surface 158 is opposite to the first surface 156 and faces away from tissue. The transparent backing material 154 can also be malleable or shaped to conform to a surface for measuring deflection of that surface.

Variations of the transparent backing materials include, but are not limited to: silicone rubber, polycarbonate, PDMS, polyethylene terephthalate, polyethylene, PMMA, gelatin, hydrogel, polymer-dispersed liquid crystal, amorphous copolyester, polyvinyl chloride, cyclic olefin copolymers, ionomer resin, polypropylene, fluorinated ethylene propylene, styrene methyl methacrylate. The first/second polymer materials: same as above and their composites or nanocomposites by adding nanomaterials made of titanium oxides, silicon oxides, cavities, or others

FIG. 3B shows components of another variation of a force detecting device 150 where a second or reference elastomer 160 is positioned adjacent to a first elastomer 152. The second elastomer 160 can be spaced from the first elastomer 152 or positioned in contact with the first elastomer 152. Optionally, an opaque barrier (e.g., a film, coating, layer, etc.) 165 is positioned between the first elastomer 152 and second elastomer 160 to enable separate measurement of the light transmission properties of each elastomer.

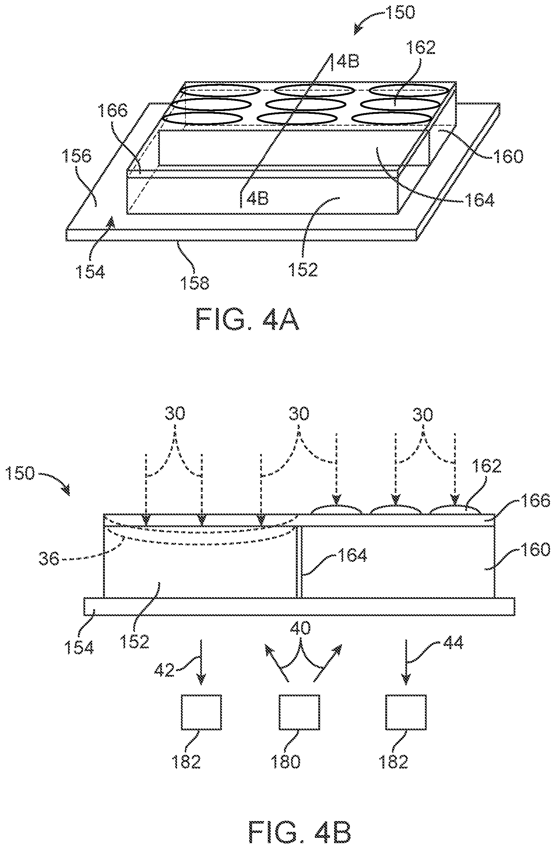

FIG. 4A illustrates another variation of components of a variation of a force detecting device 150 showing a first elastomer 152 and a second elastomer 160 on a transparent backing material 154 where the elastomers are separated by an opaque divider 164. This variation also includes an opaque cover 166 that prevents undesirable illumination from the tissue surface of the device 150. As discussed above, the second elastomer 160 includes a stiffening reinforcement or layer 162 to prevent deformation of the second elastomer 160.

FIG. 4B illustrates a cross sectional view of the components shown in FIG. 4A taken along the line 4B-4B to demonstrate an operation of a variation of a force detecting device 150. As discussed herein, a surface of the device 150 opposite to the transparent backing material 154 is positioned adjacent to a surface to be monitored. In one example, the surface being monitored is a tissue region having an artery, where the tissue region experiences displacement arising from the pressure differential caused by flow of blood in the artery. The displacement creates a force 30 that acts on a surface of the device 150. The first elastomeric material 152 experiences deformation (along with any opaque layer 166) depicted by deformed lines 36. In contrast, the second elastomer 160 is configured to resist deformation typically by the use of a stiffening layer 162 (alternative means of stiffening the polymer without affecting the light transmission properties of the elastomer are within the scope of this disclosure.)

FIG. 4B depicts a force 30 acting on the second elastomer 160 but failing to cause displacement of the second elastomer 160. FIG. 4B also shows a representation of an emitting device 180 (e.g. laser, LED, or other illumination source) that directs electromagnetic radiation (e.g., visible light or other electromagnetic radiation) through the transparent backing material 154 to the first and second elastomers 150 160. Although the figure illustrates a single emitter 180 any number of emitters can be used. The device 150 also includes one or more detectors 182 (e.g., detectors configured to measure reflected light and/or radiation). The deformation of the first elastomer 152 changes the optical properties of the elastomer 152 such that an absorption of the light (or other radiation) changes. Therefore, the reflected illumination 42 from the first elastomer 152 will be different than a reflected illumination 44 from the second non-deformed elastomer 160. This change in reflected illumination is used to determine the force 30 applied to the device 150, which is then used to determine a blood pressure of the artery causing the displacement 30. As shown, the elastomers 152 160 can be optically separated by an opaque divider 164 or via any other structural configuration that optically separates the elastomers.

Although the above example illustrates a second elastomer or reference elastomer, variations of the device 150 can omit this reference elastomer and determine an applied force by monitoring changes in a single elastomer.

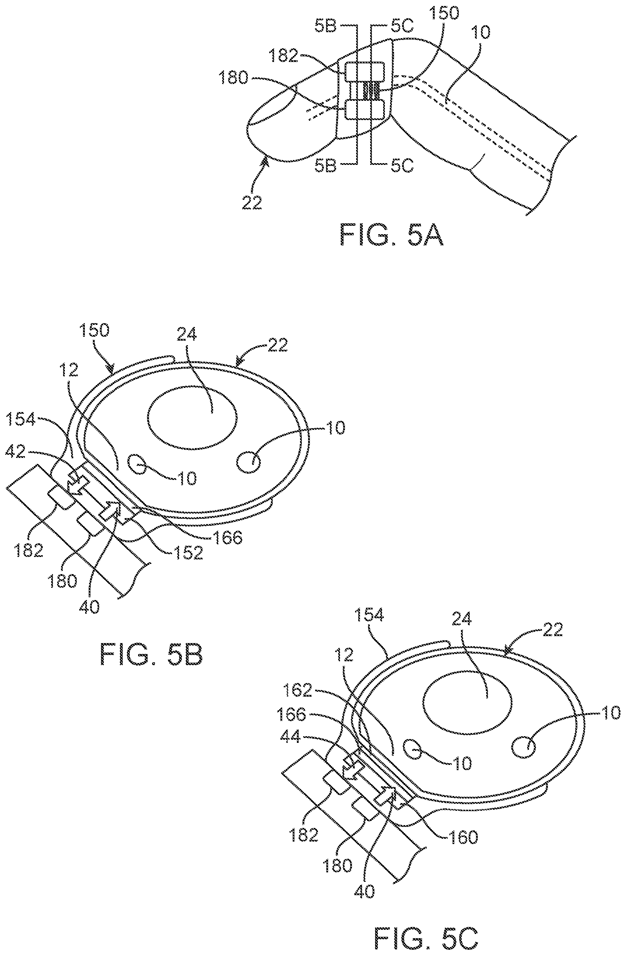

FIG. 5A illustrates a variation of a force detecting device 150 positioned on a finger 22 to detect a blood pressure of a vessel 10 within the finger 22 using displacement of tissue adjacent to the blood vessel. The illustrated variation of the device 150 includes one or more light emitters 180 and one or more light detectors 182. FIG. 5B shows a cross sectional view of the device of FIG. 5A taken along line 5B-5B. This cross sectional view illustrates a variation of a device for detecting movement 150 as the transparent backing layer 154 is shaped or shapeable to conform to a finger 22. Accordingly, the elastomeric polymer 152 and opaque barrier layer 166 conform to tissue 12 that is adjacent to an artery 10 within the finger 22. In this variation, the two vessels 10 run along a bone 24 within the finger. Pulsatile flow of blood within the vessel 10 causes displacement of tissue 12, which produces deflection of the barrier layer 166 and first elastomer 152. As discussed herein, the optical properties of the elastomer 152 change upon deflection of the elastomer 152. Therefore, light 40 emitted from an illumination component 180 is absorbed by the compressed/displaced elastomer 152 and is reflected 42 to a light detector 182. The detector 182 can produce a signal that is then used to determine a force applied to the device 150 for calculation of blood pressure within the artery 10.

FIG. 5C is a cross sectional illustration taken along lines 5C-5C from FIG. 5A. As shown, the second elastomeric polymer 160, opaque barrier layer 166, and stiffening layer 162 conform to tissue 12 that is adjacent to the artery 10 within the finger 22. The pulsatile flow of blood within the vessel 10 causes displacement of tissue 12 but fails to produce deflection of the barrier layer 166 and second elastomer 160 because of the reinforcement or stiffening layer 162. Therefore, the optical properties of the second elastomer 160 do not change because there is no deflection of the elastomer 162 (alternatively, the deflection of the second elastomer 160 is insignificant). Therefore, light 40 emitted from the illumination component 180 is absorbed by the second elastomer 160 and is reflected 44 to a light detector 182 for use as a reference for comparison for the light reflected 42 from the first elastomer. Again, the detector 182 can produce a signal that is then used to determine a force applied to the device 150 for calculation of blood pressure within the artery 10.

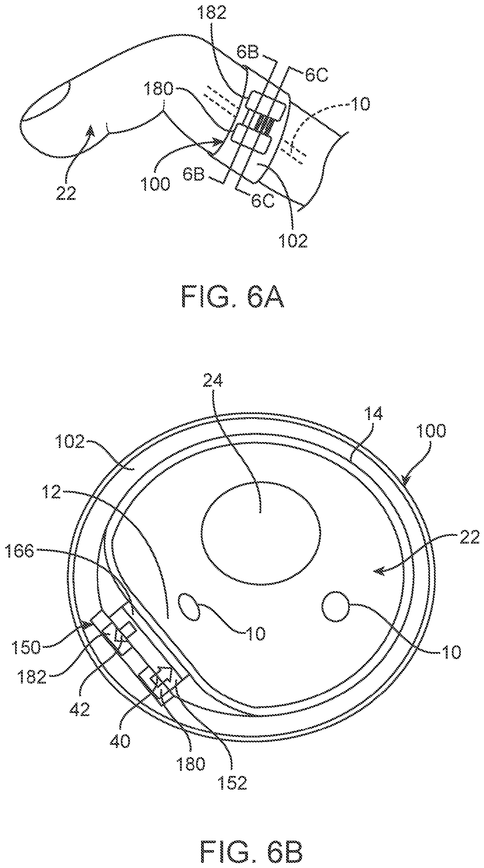

FIGS. 6A to 6D illustrate another variation of a device 100 having a force detecting apparatus positioned on a ring body 102. As shown in FIG. 6A, the device 100 includes a body structure 102 that houses the force detecting apparatus as well as an emitting component 180 and a detecting component 182.

FIG. 6B illustrates a cross sectional view of the device 100 and finger 22 taken along the line 6B-6B of FIG. 6A. This sectional view illustrates the finger 22 having a bone 24 adjacent to two vessels where the ring body 102 is positioned such that the force detecting apparatus 150 is positioned adjacent to an artery 10 and where flow of blood in the artery 10 displaces adjacent tissue 12 causing movement at the tissue surface interface of the force detecting device 150. Similar to the variations discussed above, the force detecting device 150 includes an opaque cover 166 that is placed adjacent to a skin 14 of the finger 22. A first elastomer 152 is positioned adjacent to the cover 166 with an emitter 180 of electromagnetic radiation positioned adjacent to the elastomer 152 to provide electromagnetic radiation (e.g., light) to the first elastomer 152. As noted above, deformation of the first elastomer 152 occurs as a result of tissue 12 movement caused by blood flow in artery 10. The deformation of the first elastomer 152 alters optical properties of the elastomer 152 causing the elastomer 152 to change absorption of the electromagnetic radiation. This reflected illumination 42 is then used to determine a pressure acting upon the device 150 to determine a blood pressure within the artery 10.

As shown in FIG. 6B, the force detecting device 150 does not require an optically transparent backing material. Optionally, an optically transparent backing material can be used adjacent to the elastomer 152 and within the body 102 of the device.

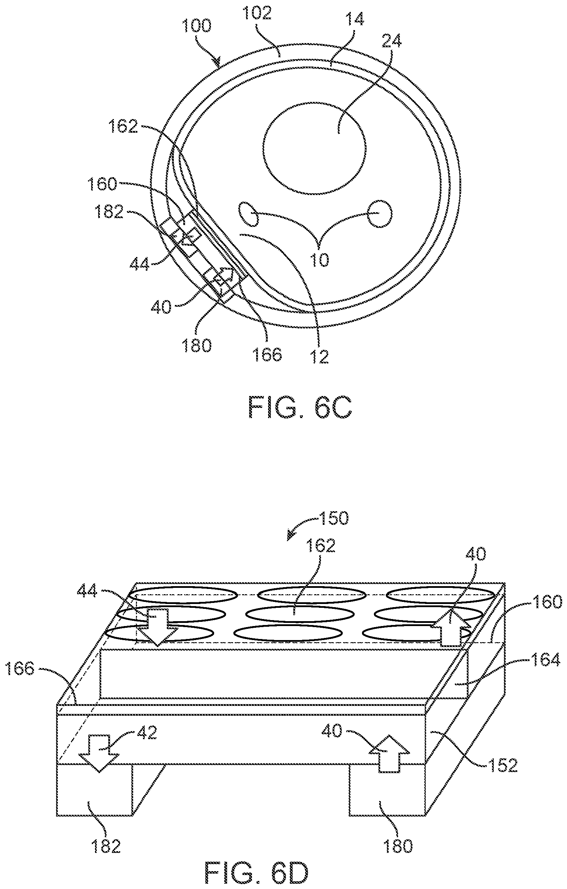

FIG. 6C illustrates a cross sectional view of the device 100 and finger 22 taken along the line 6C-6C of FIG. 6A. This sectional view also illustrates the finger 22 with a bone 24 adjacent to two vessels where the ring body 102 is positioned such that the force detecting apparatus 150 is positioned adjacent to an artery 10 and where flow of blood in the artery 10 displaces adjacent tissue 12 causing movement at the tissue surface interface of the force detecting device 150. Again, as noted herein, the second elastomer 160 comprises a stiffening layer 162 (or is otherwise reinforced to prevent deformation). As a result, the displacement of tissue 12 and skin 14 does not affect the second elastomer (or only compresses the second elastomer an insignificant amount). The emitter 180 of electromagnetic radiation is positioned adjacent to the elastomer 160 to provide electromagnetic radiation 40 (e.g., light). Since the second elastomer 160 does not deform there is no change in any optical properties of the elastomer 160. Therefore, the reflected radiation or light 44 can be used as a reference relative to light reflected from the first elastomer. The reflected radiation 42 is then used to determine a pressure acting upon the device 150 to determine a blood pressure within the artery 10.

FIG. 6D illustrates a variation of a force detecting device 150 used in FIGS. 6A to 6C. As shown, the device 150 does not require the use of a transparent backing material. Instead, the first elastomer 152 and the second elastomer 160 can be positioned adjacent to electromagnetic radiation emitters 180 and detectors 182. Therefore, the reflected radiation 42 from the first elastomer 152 and the reflected radiation 44 from the second elastomer 160 can be detected by the detector element 182 and used to ultimately determine a blood pressure of a vessel (or other force applied on the device 150).

Well-known structures, materials, or operations are not shown or described in detail to avoid obscuring aspects of the described devices. As those skilled in the art would realize, the described embodiments may be modified in various different ways, all without departing from the scope of the present invention.

Therefore, it should be appreciated that the embodiments described herein are not intended to be exhaustive of all possible embodiments in accordance with the present disclosure, and that additional embodiments may be conceived based on the subject matter disclosed herein. The scope of the invention is defined by the claims.

## Claims

1. A device for detecting a force in a surface region of tissue, the device comprising:
a transparent backing material (154) comprising a planar shape, the transparent backing material (154) comprising a first surface (156) and a second surface (158) on an opposite side of the planar shape;
a first elastomer (152) on the first surface (156) of the transparent backing material (154), where a light transmission property of the first elastomer (152) changes upon application of force to the first elastomer (152);
a second elastomer (160) on the first surface (156) of the transparent backing material (154), where a light transmission property of the second elastomer (160) changes upon application of force to the first elastomer (152); and
a stiffening layer (162) on the second elastomer (160) on a side opposite to the transparent backing material (154),
wherein when positioned on the surface region of tissue, the force in the surface region causes a deformation of the first elastomer (152) resulting in a change in the light transmission property of the first elastomer (152) and wherein when positioned on the surface region, the stiffening layer (162) prevents at least a portion of the force from changing the light transmission property of the second elastomer (160) such that the second elastomer (160) provides a reference to determine a deformation of the first elastomer (152).

2. The device of claim 1, further comprising an opaque divider (164) between the first elastomer (152) and the second elastomer (160) to block propagation of light therebetween.

3. The device of claim 2, further comprising an opaque cover (166) on the first elastomer (152) and the second elastomer (160) located on the side opposite to the transparent backing material (154), where the stiffening layer (162) is located on the opaque cover (166) and adjacent to the second elastomer (160).

4. The device of claim 2, further comprising an opaque cover (166) on the first elastomer (152) and the second elastomer (160) located on the side opposite to the transparent backing material (154), where the stiffening layer (162) is located on the second elastomer (160) and the opaque divider (164) and adjacent to the opaque cover (166).

5. The device of claim 2, further comprising a light emitting source (180) and a light detecting element (182) both located adjacent to the first elastomer (152) and to the second elastomer (160), where the light emitting source (180) is configured to illuminate the first elastomer (152) and the second elastomer (160) and where the light detecting element (182) is configured to determine an absorption of light in the first elastomer (152) and in the second elastomer (160).

6. The device of claim 5, where the light detecting element (182) is configured to transmit a signal to a controller, where the signal comprises data of the absorption of light in the first elastomer (152) and in the second elastomer (160) to determine the force in the surface region.

7. The device of claim 1, further comprising a ring body housing (102) configured to fit on a digit of an individual.

8. The device of claim 1, further comprising a ring body housing (102) configured to fit on a wrist or arm of an individual.

## Patentansprüche

1. Vorrichtung zum Erfassen Kraft einer in einem Oberflächenbereich von Gewebe, wobei die Vorrichtung aufweist:
ein transparentes Trägermaterial (154), umfassend eine ebene Form, wobei das transparente Trägermaterial (154) eine erste Oberfläche (156) und eine zweite Oberfläche (158) auf einer gegenüberliegenden Seite der ebenen Form aufweist;
ein erstes Elastomer (152) auf der ersten Oberfläche (156) des transparenten Trägermaterials (154), wobei sich eine Lichttransmissionseigenschaft des ersten Elastomers (152) bei Krafteinwirkung auf das erste Elastomer (152) ändert;
ein zweites Elastomer (160) auf der ersten Oberfläche (156) des transparenten Trägermaterials (154), wobei sich eine Lichttransmissionseigenschaft des zweiten Elastomers (160) bei Krafteinwirkung auf das erste Elastomer (152) ändert; und
eine Versteifungsschicht (162) auf dem zweiten Elastomer (160) auf einer Seite, die dem transparenten Trägermaterial (154) gegenüberliegt,
wobei, wenn diese auf dem Oberflächenbereich von Gewebe positioniert ist, die Kraft in dem Oberflächenbereich eine Verformung des ersten Elastomers (152) bewirkt, was zu einer Änderung der Lichttransmissionseigenschaft des ersten Elastomers (152) führt, und wobei, wenn diese auf dem Oberflächenbereich positioniert ist, die Versteifungsschicht (162) mindestens einen Teil der Kraft daran hindert, die Lichttransmissionseigenschaft des zweiten Elastomers (160) zu ändern, so dass das zweite Elastomer (160) eine Referenz zum Bestimmen einer Verformung des ersten Elastomers (152) bereitstellt.

2. Vorrichtung nach Anspruch 1, ferner umfassend einen undurchsichtigen Teiler (164) zwischen dem ersten Elastomer (152) und dem zweiten Elastomer (160), um die Ausbreitung von Licht dazwischen zu blockieren.

3. Vorrichtung nach Anspruch 2, ferner umfassend eine undurchsichtige Abdeckung (166) auf dem ersten Elastomer (152) und das zweite Elastomer (160), das auf der Seite angeordnet ist, die dem transparenten Trägermaterial (154) gegenüberliegt, wobei die Versteifungsschicht (162) auf der undurchsichtigen Abdeckung (166) und benachbart zu dem zweiten Elastomer (160) angeordnet ist.

4. Vorrichtung nach Anspruch 2, ferner umfassend eine undurchsichtige Abdeckung (166) auf dem ersten Elastomer (152) und das zweite Elastomer (160), das auf der Seite angeordnet ist, die dem transparenten Trägermaterial (154) gegenüberliegt, wobei die Versteifungsschicht (162) auf dem zweiten Elastomer (160) und dem undurchsichtigen Teiler (164) und benachbart zu der undurchsichtigen Abdeckung (166) angeordnet ist.

5. Vorrichtung nach Anspruch 2, ferner umfassend eine lichtemittierende Quelle (180) und ein Lichtdetektorelement (182), die jeweils benachbart zu dem ersten Elastomer (152) und dem zweiten Elastomer (160) angeordnet sind, wobei die lichtemittierende Quelle (180) so konfiguriert ist, dass sie das erste Elastomer (152) und das zweite Elastomer (160) beleuchtet und wobei das Lichtdetektorelement (182) so konfiguriert ist, dass es eine Absorption von Licht in dem ersten Elastomer (152) und in dem zweiten Elastomer (160) bestimmt.

6. Vorrichtung nach Anspruch 5, wobei das Lichtdetektorelement (182) so konfiguriert ist, dass es ein Signal an eine Steuerung überträgt, wobei das Signal Daten über die Absorption von Licht in dem ersten Elastomer (152) und in dem zweiten Elastomer (160) umfasst, um die Kraft in dem Oberflächenbereich zu bestimmen.

7. Vorrichtung nach Anspruch 1, ferner umfassend ein Ringkörpergehäuse (102), das so konfiguriert ist, dass es auf einen Finger einer Person passt.

8. Vorrichtung nach Anspruch 1, ferner umfassend ein Ringkörpergehäuse (102), das so konfiguriert ist, dass es auf ein Handgelenk oder einen Arm einer Person passt.

## Revendications

1. Dispositif de détection d'une force dans une région de surface de tissu, le dispositif comprenant :
un matériau de renfort transparent (154) comprenant une forme plane, le matériau de renfort transparent (154) comprenant une première surface (156) et une seconde surface (158) sur un côté opposé de la forme plane ;
un premier élastomère (152) sur la première surface (156) du matériau de renfort transparent (154), où une propriété de transmission de lumière du premier élastomère (152) change lors de l'application de force sur le premier élastomère (152) ;
un second élastomère (160) sur la première surface (156) du matériau de renfort transparent (154), où une propriété de transmission de lumière du second élastomère (160) change lors de l'application de force sur le premier élastomère (152) ; et
une couche de raidissement (162) sur le second élastomère (160) sur un côté opposé au matériau de renfort transparent (154),
dans lequel lorsqu'elle est positionnée sur la région de surface de tissu, la force dans la région de surface provoque une déformation du premier élastomère (152) résultant en un changement de la propriété de transmission de lumière du premier élastomère (152) et dans lequel lorsqu'elle est positionnée sur la région de surface, la couche de raidissement (162) empêche au moins une partie de la force de changer la propriété de transmission de lumière du second élastomère (160) de sorte que le second élastomère (160) fournisse une référence pour déterminer une déformation du premier élastomère (152).

2. Dispositif selon la revendication 1, comprenant en outre un diviseur opaque (164) entre le premier élastomère (152) et le second élastomère (160) pour bloquer la propagation de lumière entre eux.

3. Dispositif selon la revendication 2, comprenant en outre un couvercle opaque (166) sur le premier élastomère (152) et le second élastomère (160) situé sur le côté opposé au matériau de renfort transparent (154), où la couche de raidissement (162) est située sur le couvercle opaque (166) et adjacente au second élastomère (160).

4. Dispositif selon la revendication 2, comprenant en outre un couvercle opaque (166) sur le premier élastomère (152) et le second élastomère (160) situé sur le côté opposé au matériau de renfort transparent (154), où la couche de raidissement (162) est située sur le second élastomère (160) et le diviseur opaque (164) et adjacente au couvercle opaque (166).

5. Dispositif selon la revendication 2, comprenant en outre une source d'émission de lumière (180) et un élément de détection de lumière (182) tous deux situés adjacents au premier élastomère (152) et au second élastomère (160), où la source d'émission de lumière (180) est configurée pour illuminer le premier élastomère (152) et le second élastomère (160) et où l'élément de détection de lumière (182) est configuré pour déterminer une absorption de lumière dans le premier élastomère (152) et dans le second élastomère (160).

6. Dispositif selon la revendication 5, où l'élément de détection de lumière (182) est configuré pour transmettre un signal à un dispositif de commande, où le signal comprend des données de l'absorption de lumière dans le premier élastomère (152) et dans le second élastomère (160) pour déterminer la force dans la région de surface.

7. Dispositif selon la revendication 1, comprenant en outre un logement de corps annulaire (102) configuré pour s'ajuster sur un doigt d'un individu.

8. Dispositif selon la revendication 1, comprenant en outre un logement de corps annulaire (102) configuré pour s'ajuster sur un poignet ou bras d'un individu.
